# FASCICULE DE BREVET EUROPEEN

(11) **EP 3 254 101 B1**
(45) Date de publication et mention de la délivrance du brevet: **03.04.2024**
(21) Numéro de dépôt: 16706980.6
(22) Date de dépôt: 05.02.2016
(51) Int. Cl.: G01N 33/28, G01N 33/30

(54) **INSTALLATION ET PROCÉDÉ DE SUIVI DE L'ÉVOLUTION DE LA BASICITÉ D'UN LUBRIFIANT**
ANLAGE UND VERFAHREN ZUR ÜBERWACHUNG DER ÄNDERUNG DER BASIZITÄT EINES SCHMIERMITTELS
FACILITY AND METHOD FOR MONITORING THE VARIATION IN THE BASICITY OF A LUBRICANT

(30) Priorité: 06.02.2015 FR 1550956
(43) Date de publication de la demande: 13.12.2017
(73) Titulaire: TotalEnergies OneTech, 92400 Courbevoie (FR)
(72) Inventeur: ROMAN, Jean Philippe, 38780 Septeme (FR); AMIOT, Arnaud, 92500 Rueil Malmaison (FR); CHAUDOREILLE, François, 73100 Gresy sur Aix (FR); ADJALI, Mustapha, 73100 Aix Les Bains (FR); JUSTON, Raphael, 73000 Chambéry (FR)
(74) Mandataire: Lavoix
(86) Numéro de dépôt international: PCT/EP2016/052451
(87) Numéro de publication internationale: WO 2016/124721

(56) Documents cités:
- WO-A1-2010/046591
- AU-A1- 2005 201 261
- CN-U- 202 421 093
- US-A1- 2002 149 768
- US-A1- 2007 084 271
- US-A1- 2014 146 307
- JOSÉ SALGUEIRO ET AL: "On-line Oil Monitoring and Diagnosis", STROJNISKI VESTNIK - JOURNAL OF MECHANICAL ENGINEERING, vol. 10, no. 59, 15 octobre 2013 (2013-10-15), pages 604-612, XP055208567, ISSN: 0039-2480, DOI: 10.5545/sv-jme.2013.973
- Alberto Villar ET AL: "Chemometric methods applied to the calibration of a Vis-NIR sensor for gas engine's condition monitoring", Analytica Chimica Acta, vol. 705, no. 1-2, 1 October 2011 (2011-10-01), pages 174-181, XP055208678, ISSN: 0003-2670, DOI: 10.1016/j.aca.2011.04.048

## Description

La présente invention a trait à une installation de suivi de l'évolution de la basicité d'un lubrifiant circulant dans un équipement, tel qu'un moteur de navire. L'invention a également trait à un procédé de suivi de l'évolution de la basicité d'un lubrifiant.

Dans le domaine des moteurs à combustion interne utilisés sur les navires de commerce, il est connu qu'il convient de suivre la situation d'un moteur en analysant un lubrifiant circulant dans ce moteur. Une telle analyse permet de détecter des phénomènes d'usure ou de corrosion qui ont tendance à se produire dans un moteur. Par le passé, le fonctionnement des moteurs était relativement stabilisé et il suffisait de contrôler la qualité d'un lubrifiant de façon ponctuelle, lors des escales, pour anticiper les opérations de maintenance à réaliser. De nos jours, les moteurs sont de plus en plus élaborés et sensibles aux phénomènes d'usure ou de corrosion, de sorte que des analyses doivent être réalisées en mer, notamment pour suivre l'indice de basicité ou BN (de l'Anglais « Base Number ») de l'huile moteur. Ceci impose de former le personnel et d'embarquer un matériel élaboré, dont le fonctionnement est relativement difficile à maitriser, même par un matelot formé. En outre, ceci augmente la charge de travail du personnel.

Dans ce cadre, il est connu de l'article « A low cost mid-infrared sensor for on line contamination monitoring of lubricating oils in marine engines » de Ben Mohammadi et al. (Optical Sensing and Détection Conférence - Bruxelles - 12-15/4/2010) de prévoir un système d'analyse du TBN (ou « Total Base Number ») qui correspond à l'indice de basicité total, au moyen d'un capteur dans lequel est disposé un échantillon du lubrifiant à étudier. Le matériel utilisé est élaboré et sa manipulation est complexe. Si un tel matériel devait être embarqué sur un navire, sa mise en oeuvre nécessiterait de prélever régulièrement sur le moteur du navire une quantité d'huile destinée à constituer un échantillon de mesure. Ceci serait à la fois long et complexe.

WO-A-03/073075 divulgue une méthode d'analyse de la basicité d'un lubrifiant au cours de laquelle une mesure, effectuée sur un échantillon d'un lubrifiant à contrôler, est comparée à des mesures effectuées sur des échantillons de lubrifiant de référence. Là encore, cette démarche est prévue pour un fonctionnement en laboratoire et requiert une main d'oeuvre qualifiée.

WO-A-2010/046591 prévoit d'utiliser un système embarqué dans lequel l'huile sortant d'un moteur est dirigé vers un composant fonctionnel associé à un système de mesure permettant de déterminer son indice de basicité. En pratique, le débit d'huile sortant du moteur est faible et l'écoulement en sortie du moteur est constitué de gouttelettes qui ruissèlent à l'intérieur d'une canalisation, au point qu'il n'est pas certain que le composant fonctionnel soit alimenté avec un débit d'huile suffisant pour que les mesures qu'il effectue soient correctes.

US-A-2007/0084271 enseigne de déterminer l'indice de basicité d'un lubrifiant au moyen d'un générateur de signal couplé à un capteur de courant. Compte tenu des matériels utilisés, cette approche est complexe à mettre en oeuvre et sensible à des perturbations.

Ces problèmes se posent non seulement sur les moteurs deux temps ou quatre temps de propulsion des navires mais également sur d'autres moteurs secondaires également embarqués sur des navires, par exemple pour des accessoires de type palan. De tels problèmes se posent également pour des boîtes à vitesse de matériels embarqués sur des navires ou d'installations fixes, telle qu'une hydrolienne ou une éolienne. De façon générale, la surveillance de la basicité d'un lubrifiant est importante pour tous les équipements lubrifiés et les techniques connues sont peu propices à une automatisation.

Il est également connu de AU-A-2005201261 de monter un système multi-capteur entre une ligne de circulation d'huile et un bac de récupération, ce système comprenant, entre autres, un réservoir de refroidissement disposé en aval d'une vanne principale et d'une vanne de calibration et en amont d'un capteur à fluorescence X comprenant un tube dans lequel s'écoule l'huile à analyser. Ce matériel est complexe et délicat à faire fonctionner

C'est à ces inconvénients qu'entend plus particulièrement remédier l'invention en proposant une nouvelle installation de suivi de l'évolution de la basicité d'un lubrifiant circulant dans un équipement qui est adapté pour fonctionner de façon simple et autonome, ce qui libère notamment le personnel embarqué à bord d'un navire de tâches répétitives et élaborées.

A cet effet, l'invention concerne une installation de suivi de l'évolution de la basicité d'un lubrifiant circulant dans un équipement, cette installation comprenant au moins une conduite de circulation du lubrifiant, cette conduite étant raccordée, en amont, à l'équipement en question et, en aval, à un bac de récupération de lubrifiant, ainsi qu'au moins un capteur de détermination de l'indice de basicité du lubrifiant. L'installation comprend, en outre, une première vanne d'interruption commandée de la circulation du lubrifiant dans la conduite et un réservoir tampon d'accumulation du lubrifiant. L'installation comprend une première ligne de dérivation raccordée d'une part à la conduite en amont de la première vanne, et d'autre part au réservoir tampon, une deuxième vanne d'interruption commandée de la circulation du lubrifiant dans la première ligne de dérivation, une deuxième ligne d'évacuation du lubrifiant, du réservoir tampon vers le bac de récupération, cette deuxième ligne étant disposée en aval de la première ligne de dérivation, et une troisième vanne d'interruption commandée de la circulation du lubrifiant dans la deuxième ligne d'évacuation. En outre, le capteur est disposé sur la deuxième ligne d'évacuation et permet de déterminer l'indice de basicité du lubrifiant en sortie du réservoir tampon.

Grâce à l'invention, le réservoir tampon est utilisé pour accumuler une quantité de lubrifiant suffisante pour permettre d'alimenter correctement le capteur d'indice de basicité.

Le lubrifiant dont il est question dans la présente invention comprend au moins une huile de base lubrifiante. En général, les huiles de base lubrifiantes peuvent être des huiles d'origine minérales, synthétiques ou végétales ainsi que leurs mélanges. Les huiles minérales ou synthétiques généralement utilisées appartiennent à l'un des groupes I à V selon les classes définies dans la classification API (ou leurs équivalents selon la classification ATIEL) telle que résumée ci-dessous. La classification APl est définie dans American Petroleum Institute 1509 "Engine oil Licensing and Certification System" 17ieme édition, Septembre 2012. La classification ATIEL est définie dans "The ATIEL Code of Practice", numéro 18, Novembre 2012.

| | Teneur en saturés | Teneur en soufre | Indice de viscosité |
|---|---|---|---|
| Groupe I Huiles minérales | < 90 % | > 0,03 % | 80 ≤ VI < 120 |
| Groupe II Huiles hydrocraquées | ≥ 90 % | ≤ 0,03 % | 80 ≤ VI < 120 |
| Groupe III Huiles hydrocraquées ou hydro-isomérisées | ≥ 90 % | ≤ 0,03 % | ≥ 120 |
| Groupe IV | PAO (Poly alpha olefins) | | |
| Groupe V | Esters et autres bases non incluses dans bases groupes I à IV | | |

Les huiles minérales de Groupe I peuvent être obtenues par distillation de bruts naphténiques ou paraffiniques sélectionnés puis purification des distillats obtenus par des procédés tels que extraction par solvant, déparaffinage au solvant ou catalytique, hydrotraitement ou hydrogénation. Les huiles des Groupes Il et III sont obtenues par des procédés de purification plus poussés, par exemple une combinaison de traitement choisi parmi l'hydrotraitement, l'hydrocraquage, l'hydrogénation et le déparaffinage catalytique. Des exemples d'huiles de base synthétiques de Groupe IV et V incluent les polyisobutènes, les alkylbenzènes et les poly-alphas oléfines telles que les polybutènes ou encore les esters.

Dans les lubrifiants, les huiles de base lubrifiantes peuvent être utilisées seules ou en mélange. Par exemple, une huile minérale peut être combinée avec une huile synthétique.

Les huiles cylindres pour moteurs marins deux temps sont généralement caractérisées par un grade viscosimétrique SAE-40 à SAE-60, généralement SAE-50 équivalent à une viscosité cinématique à 100°C comprise entre 16,3 et 21,9 mm²/s mesurée selon la norme ASTM D445. Les huiles de grade SAE-40 ont une viscosité cinématique à 100°C comprise entre 12,5 et 16,3 cSt mesurée selon la norme ASTM D445. Les huiles de grade SAE-50 ont une viscosité cinématique à 100°C comprise entre 16,3 et 21,9 cSt mesurée selon la norme ASTM D445. Les huiles de grade SAE-60 ont une viscosité cinématique à 100°C comprise entre 21,9 et 26,1 cSt mesurée selon la norme ASTM D445. Les lubrifiants utilisés avec l'invention ont, de préférence, une viscosité cinématique mesurée selon la norme ASTM D445 à 100°C allant de 12,5 à 26,1 cSt, préférentiellement de 16,3 à 21,9 cSt. Pour obtenir de telle viscosité, ces lubrifiants peuvent comprendre en outre un ou plusieurs additifs. Typiquement, une formulation classique de lubrifiant pour moteurs marins, de préférence deux temps, est de grade SAE-40 à SAE-60, préférentiellement SAE-50 (selon la classification SAE J300) et comprend au moins 40 % en poids d'huile de base lubrifiante d'origine minérale, synthétique ou leurs mélanges, adaptée à l'utilisation pour un moteur marin. Par exemple, une huile de base lubrifiante de groupe I, selon la classification API, peut être utilisée pour la formulation d'un lubrifiant cylindre. Les huiles de base lubrifiantes de groupe I ont un Indice de Viscosité (VI) allant de 80 à 120 ; leur teneur en soufre est supérieure à 0,03 % et leur teneur en composés hydrocarbonés saturés est inférieure à 90 %.

Le lubrifiant peut comprendre en outre un additif choisi parmi les détergents surbasés ou les détergents neutres. Les détergents sont typiquement des composés anioniques comportant une longue chaîne hydrocarbonée lipophile et une tête hydrophile, le cation associé est typiquement un cation métallique d'un métal alcalin ou alcalino-terreux. Les détergents sont préférentiellement choisis parmi les sels de métaux alcalins ou alcalino-terreux (notamment préférentiellement le calcium, le magnésium, le sodium ou le baryum) d'acides carboxyliques, sulfonates, salicylates, naphténates, ainsi que les sels de phénates. Ces sels métalliques peuvent contenir le métal en quantité approximativement stoechiométrique par rapport au(x) groupement(s) anionique(s) du détergent. Dans ce cas, on parle de détergents non surbasés ou « neutres », bien qu'ils apportent également une certaine basicité. Ces détergents « neutres » ont typiquement un BN, mesuré selon ASTM D2896, inférieur à 150 mg KOH/g, ou inférieur à 100 mg KOH/g, ou encore inférieur à 80 mg KOH/g de détergent. Ce type de détergents dits neutres peut contribuer pour partie au BN des lubrifiants. Sont employés par exemple des détergents neutres de type carboxylates, sulfonates, salicylates, phénates, naphténates de métaux alcalins et alcalino-terreux, par exemple de calcium, sodium, magnésium, baryum. Lorsque le métal est en excès (en quantité supérieure à la quantité stoechiométrique par rapport au(x) groupements(s) anionique(s) du détergent), on a affaire à des détergents dits surbasés. Leur BN est élevé, supérieur à 150 mg KOH/g de détergent, typiquement allant de 200 à 700 mg KOH/g de détergent, préférentiellement de 250 à 450 mg KOH/g de détergent. Le métal en excès apportant le caractère surbasé au détergent se présente sous la forme de sels métalliques insolubles dans l'huile, par exemple carbonate, hydroxyde, oxalate, acétate, glutamate, préférentiellement carbonate. Dans un même détergent surbasé, les métaux de ces sels insolubles peuvent être les mêmes que ceux des détergents solubles dans l'huile ou bien être différents. Ils sont préférentiellement choisis parmi le calcium, le magnésium, le sodium ou le baryum. Les détergents surbasés se présentent ainsi sous forme de micelles composées de sels métalliques insolubles maintenues en suspension dans le lubrifiant par les détergents sous forme de sels métalliques solubles dans l'huile. Ces micelles peuvent contenir un ou plusieurs types de sels métalliques insolubles, stabilisés par un ou plusieurs types détergents. Les détergents surbasés comportant un seul type de sel métallique soluble détergent seront généralement nommés d'après la nature de la chaîne hydrophobe de ce dernier détergent. Ainsi, ils seront dits de type phénate, salicylate, sulfonate, naphténate selon que ce détergent est respectivement un phénate, salicylate, sulfonate, ou naphténate. Les détergents surbasés seront dits de type mixte si les micelles comprennent plusieurs types de détergents, différents entre eux par la nature de leur chaîne hydrophobe. Le détergent surbasé et le détergent neutre peuvent être choisis parmi les carboxylates, sulfonates, salicylates, naphténates, phénates, et les détergents mixtes associant au moins deux de ces types de détergents. Le détergent surbasé et le détergent neutre sont notamment des composés à base de métaux choisis parmi le calcium, le magnésium, le sodium ou le baryum, préférentiellement le calcium ou le magnésium. Le détergent surbasé peut être surbasé par des sels insolubles métalliques choisis dans le groupe des carbonates de métaux alcalins et alcalino-terreux, préférentiellement le carbonate de calcium. Le lubrifiant peut comprendre au moins un détergent surbasé et au moins un détergent neutre tels que définis ci-dessus.

Comme mentionné ci-dessus, dans un mode de réalisation de l'invention, le lubrifiant peut avoir un BN déterminé selon la norme ASTM D-2896 d'au plus 50, de préférence d'au plus 40, avantageusement d'au plus 30 milligrammes de potasse par gramme de lubrifiant, notamment allant de 10 à 30, de préférence de 15 à 30, avantageusement de 15 à 25 milligrammes de potasse par gramme de lubrifiant. Dans ce mode de réalisation de l'invention, le lubrifiant peut ne pas comprendre de détergents à base de métaux alcalins ou alcalino-terreux surbasés par des sels métalliques de carbonate.

Dans un autre mode de réalisation de l'invention, le lubrifiant a un BN déterminé selon la norme ASTM D-2896 d'au moins 50, préférentiellement d'au moins 60, plus préférentiellement d'au plus 70, avantageusement de 70 à 100.

Le lubrifiant peut également comprendre au moins un autre additif supplémentaire choisi parmi les dispersants, les additifs anti-usure ou tout autre additif fonctionnel. Les dispersants sont des additifs bien connus employés dans la formulation de lubrifiant, notamment pour application dans le domaine marin. Leur rôle premier est de maintenir en suspension les particules présentes initialement ou apparaissant dans le lubrifiant au cours de son utilisation dans le moteur. Ils préviennent leur agglomération en jouant sur l'encombrement stérique. Ils peuvent présenter également un effet synergique sur la neutralisation. Les dispersants utilisés comme additifs pour lubrifiant contiennent typiquement un groupement polaire, associé à une chaîne hydrocarbonée relativement longue, contenant généralement de 50 à 400 atomes de carbone. Le groupement polaire contient typiquement au moins un élément azote, oxygène ou phosphore. Les composés dérivés de l'acide succinique sont des dispersants particulièrement utilisés comme additifs de lubrification. On utilise en particulier les succinimides, obtenus par condensation d'anhydrides succiniques et d'amines, les esters succiniques obtenus par condensation d'anhydrides succiniques et d'alcools ou polyols. Ces composés peuvent être ensuite traités par divers composés notamment soufre, oxygène, formaldéhyde, acides carboxyliques et composés contenant du bore ou du zinc pour produire par exemple des succinimides boratées ou des succinimides bloqués au zinc. Les bases de Mannich, obtenues par polycondensation de phénols substitués par des groupements alkyles, de formaldéhyde et d'amines primaires ou secondaires, sont également des composés utilisés comme dispersants dans les lubrifiants. Dans un mode de réalisation de l'invention, la teneur en dispersant peut être supérieure ou égal à 0,1%, de préférence de 0,5 à 2%, avantageusement de 1 à 1,5 % en poids par rapport au poids total du lubrifiant. Les additifs anti-usure protègent les surfaces en frottement par formation d'un film protecteur adsorbé sur ces surfaces. Le plus couramment utilisé est le di thiophosphate de zinc ou DTPZn. On trouve également dans cette catégorie divers composés phosphorés, soufrés, azotés, chlorés et borés. Il existe une grande variété d'additifs anti-usure, mais la catégorie la plus utilisée est celle des additifs phospho soufrés comme les alkylthiophosphates métalliques, en particulier les alkylthiophosphates de zinc, et plus spécifiquement les dialkyldithiophosphates de zinc ou DTPZn. Les composés préférés sont de formule Zn((SP(S)(OR₁)(OR₂))₂ , ou R₁ et R₂ sont des groupements alkyl , comportant préférentiellement de 1 à 18 atomes de carbones. Le DTPZn est typiquement présent à des teneurs de l'ordre de 0,1 à 2 % en poids par rapport au poids total du lubrifiant. Les phosphates d'amines, les polysulfures, notamment les oléfines soufrées, sont également des additifs anti-usure employés couramment. On rencontre également usuellement dans les lubrifiants pour moteur marin des additifs anti-usure et extrême pression de type azotés et soufrés, tels que par exemple les dithiocarbamates métalliques, en particulier dithiocarbamate de molybdène. Les esters du glycérol sont également des additifs anti-usure. On peut citer par exemple les mono, di et trioléates, monopalmitates et monomyristates. Dans un mode de réalisation, la teneur en additifs anti-usure va de 0,01 à 6 %, préférentiellement de 0,1 à 4 % en poids par rapport au poids total du lubrifiant.

Les autres additifs fonctionnels peuvent être choisis parmi les agents épaississants, les additifs anti-mousse pour contrer l'effet des détergents, pouvant être par exemple des polymères polaires tels que polyméthylsiloxanes, polyacrylates, les additifs anti-oxydant et/ou anti rouille, par exemple détergents organo-métalliques ou thiadiazoles. Ceux-ci sont connus de l'homme du métier. Ces additifs sont généralement présents à une teneur en poids de 0,1 à 5% par rapport au poids total du lubrifiant.

Selon des aspects avantageux mais non obligatoires, une installation conforme à l'invention peut incorporer une ou plusieurs des caractéristiques suivantes, prises dans toute combinaison techniquement admissible :
- L'installation comprend des moyens de mise sous pression gazeuse du volume intérieur du réservoir tampon.
- Les moyens de mise sous pression gazeuse comprennent une source d'air comprimé et un jeu de vannes ou un distributeur pneumatique de mise en communication sélective du volume intérieur du réservoir tampon avec la source d'air comprimé ou l'atmosphère ambiante.
- L'installation comprend des moyens de détection du niveau de lubrifiant dans le réservoir tampon.
- Les moyens de détection du niveau de lubrifiant dans le réservoir comprennent un capteur de la pression gazeuse dans le volume intérieur du réservoir tampon.
- L'installation comprend, en outre, un capteur de densité, viscosité, humidité et température disposé également sur la deuxième ligne d'évacuation, ainsi qu'un capteur de la teneur en fer dissout du lubrifiant présent dans le réservoir tampon.

Par ailleurs, l'invention concerne un procédé automatisé de suivi de l'évolution de la basicité d'un lubrifiant circulant dans un équipement, au moyen d'une installation telle que mentionnée ci-dessus. Ce procédé comprend des étapes consistant à :
a) fermer la première vanne
b) ouvrir la deuxième vanne et fermer la troisième vanne pour alimenter le réservoir tampon à partir d'une quantité de lubrifiant accumulée dans la conduite, en amont de la première vanne
c) ouvrir la troisième vanne pour faire circuler le lubrifiant présent dans le réservoir tampon à travers la deuxième ligne d'évacuation, au contact du capteur de détermination de l'indice de basicité du lubrifiant
d) utiliser un signal de sortie de ce capteur pour déterminer l'indice de basicité du lubrifiant.

De façon avantageuse, un tel procédé peut incorporer une ou plusieurs des caractéristiques suivantes, prises dans toute combinaison techniquement admissible :
- Lorsque l'installation comprend des moyens de mise sous pression gazeuse du contenu du réservoir tampon, il est prévu une étape e) postérieure à l'étape b) et antérieure à l'étape c) et consistant à mettre sous pression gazeuse le réservoir tampon, avec une pression comprise entre 6 et 12 bars, de préférence entre 7 et 10 bars, de préférence encore égale à 7 bars.
- L'étape c) est interrompue alors qu'une quantité résiduelle de lubrifiant demeure dans le réservoir tampon
- Le procédé comporte une étape f) postérieure à l'étape d) et consistant à décolmater un filtre intégré à la première ligne de dérivation, en faisant circuler du lubrifiant du réservoir tampon vers la conduite.

L'invention concerne également une méthode de suivi du fonctionnement d'un équipement embarqué sur un navire, cette méthode comprenant la détermination, à bord du navire, de l'indice de basicité d'un lubrifiant de l'équipement en question par la mise en oeuvre d'un procédé automatisé tel que mentionné ci-dessus.

L'invention sera mieux comprise et d'autres avantages de celle-ci apparaitront plus clairement à la lumière de la description qui va suivre de trois modes de réalisation d'une installation conforme à son principe, donnée uniquement à titre d'exemple et faite en référence aux dessins annexés dans lesquels :
- la figure 1 est une représentation schématique de principe d'une installation conforme à l'invention telle qu'embarquée sur un navire,
- la figure 2 est une représentation schématique à plus petite échelle de la partie fluidique de l'installation de la figure 1 dans une première configuration d'utilisation,
- les figures 3 à 5 sont des vues analogues à la figure 2 lorsque l'installation est dans une deuxième, une troisième et une quatrième configurations d'utilisation,
- la figure 6 est une vue analogue à la figure 1 pour une installation conforme à un deuxième mode de réalisation de l'invention,
- les figures 7 à 11 et 13 à 18 sont des vues analogues à la figure 2 pour l'installation de la figure 6 dans différentes configurations d'utilisation,
- la figure 12 est une vue à plus grande échelle du détail XII à la figure 11, et
- la figure 19 est une vue analogue à la figure 1 pour une installation conforme à un troisième mode de réalisation de l'invention.

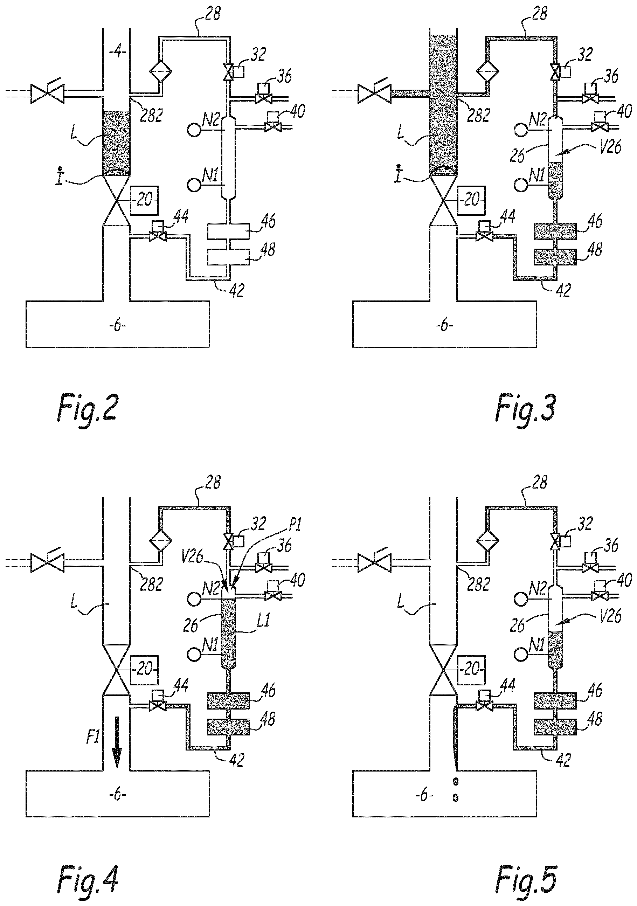

Sur les figures 2 à 5 et 7 à 18, le lubrifiant présent ou circulant dans une partie de l'installation est représenté en grisé.

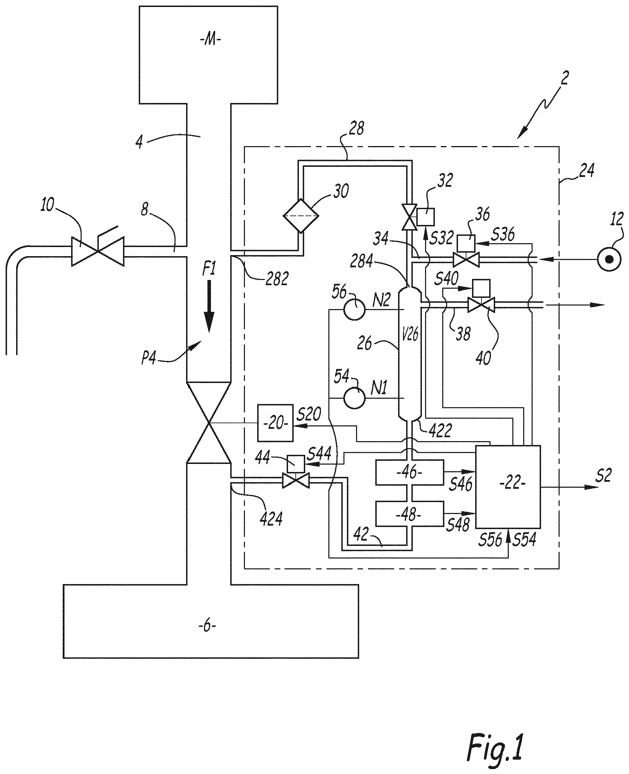

L'installation 2 représentée aux figures 1 à 5 est embarquée sur un navire représenté à la figure 1 par son moteur M qui comporte plusieurs cylindres, par exemple douze ou quatorze cylindres. Une conduite 4 relie le moteur M à un bac 6 de récupération de lubrifiant. En pratique, l'huile moteur s'écoule par gravité dans la conduite 4 avec une pression P4 comprise entre 1,1 et 6 bars absolus. Le débit d'huile dans la conduite 4 peut être faible, au point que l'huile ruisselle sur la paroi interne de cette conduite.

La conduite 4 s'étend verticalement, de haut en bas, du moteur M vers le bac 6. Dans ce mode de réalisation, l'huile s'écoulant dans la conduite 4 provient d'au moins un cylindre du moteur M.

Un piquage 8 est prévu sur la conduite 4 et équipé d'une vanne 10 commandée manuellement, ce qui permet de prélever une quantité d'huile sortant du moteur M afin de procéder à des analyses physico-chimiques, selon une approche connue en soi.

L'installation 2 comprend une vanne d'arrêt 20 montée sur la conduite 4 et qui permet d'interrompre, de façon sélective, l'écoulement d'huile dans la conduite 4, en direction du bac 6. La vanne d'arrêt 20 est commandée par une unité électronique 22 au moyen d'un signal électrique S20.

Comme visible uniquement à la figure 1, l'installation 2 comprend un boîtier 24, représenté par sa trace en trait d'axe et à l'intérieur duquel sont disposés les éléments constitutifs de l'installation 2, à l'exception de la partie de la vanne d'arrêt 20 qui est intégrée à la conduite 4.

L'installation 2 comprend également un réservoir tampon 26 qui est disposé dans le boîtier 24 et qui est relié à la conduite 4 au moyen d'une première ligne de dérivation 28.

On note 282 l'embouchure de la ligne 28. Cette embouchure est disposée en amont de la vanne 20 sur la conduite 4. La première ligne de dérivation 28 est équipée, en allant de son embouchure 282 vers son débouché 284 dans le réservoir tampon 26, d'un filtre 30, d'une vanne d'arrêt 32 et d'un piquage 34. Le filtre 30 sert à empêcher des impuretés de taille trop importante de s'écouler dans la première ligne de dérivation 28. La vanne d'arrêt 32 permet, au choix, de rendre passante ou de fermer la première ligne de dérivation 28. La vanne 32 est commandée par l'unité électronique 22, au moyen d'un signal électrique S32. Le piquage 34 est relié, à travers une vanne commandée 36, à une source 12 d'air sous pression qui ne fait pas partie de l'installation 2 mais appartient aux équipements standards d'un navire.

En pratique, la source 12 d'air sous pression peut être un compresseur embarqué sur le navire et qui alimente un réseau d'air comprimé qui sert également à d'autres équipements que l'installation 2. En variante, la source 12 peut être une pompe dédiée à l'installation 2.

L'installation 2 comprend également un piquage 38 raccordé au réservoir 26, sur lequel est montée une vanne d'arrêt 40 et qui permet de mettre en communication le volume intérieur V26 du réservoir 26 avec l'atmosphère ambiante.

Dans ce mode de réalisation, les piquages 34 et 38 sont indépendants. En variante, ils peuvent être remplacés par un unique piquage, raccordé à la première ligne 28 ou directement au réservoir 26, sur lequel les vannes 36 et 40 sont montées en parallèle, en étant respectivement reliées à la source 12 d'air sous pression et à l'atmosphère ambiante. Dans ce cas, il est possible de combiner les vannes 36 et 40 sous la forme d'une unique vanne trois voies. Les vannes 36 et 40 sont commandées par l'unité électronique 22 au moyen de signaux électriques respectifs S36 et S40.

L'installation 2 comprend également une deuxième ligne 42 d'évacuation du lubrifiant, du volume intérieur V26 du réservoir 26 vers le bac de récupération 6. La deuxième ligne d'évacuation 42 est donc disposée en aval de la première ligne de dérivation 28 et du réservoir 26, sur le trajet d'écoulement du lubrifiant. Dans l'exemple, la deuxième ligne 42 s'étend du réservoir 26 vers la conduite 4. Son embouchure 422 est située en partie basse du réservoir 26, alors que son débouché 424 est disposé sur la conduite 4, en aval de la vanne d'arrêt 20, comme représenté sur les figures, ce qui permet de réduire le temps d'un cycle d'analyse car la vanne d'arrêt 20 peut être fermée pour créer une colonne d'huile dans la conduite 4, alors que des étapes de mesure ont lieu. En variante, le débouché 424 de la deuxième ligne 42 est disposé en amont de la vanne, d'arrêt 20, ce qui permet d'exécuter simultanément des étapes de vidange et de décolmatage du filtre 30 et, éventuellement, de réduire le coût de l'installation 2.

La deuxième ligne 42 est équipée d'une vanne d'arrêt 44 qui est commandée par l'unité électronique 22 au moyen d'un signal électrique S44.

Deux capteurs 46 et 48 sont disposés sur la ligne 42, en amont de la vanne 44.

Le capteur 46 permet de mesurer la densité D, la viscosité V, l'humidité H et la température T d'un liquide présent ou s'écoulant dans la deuxième ligne 42. Ce capteur peut être du type de celui commercialisé par la société AVENISENSE sous la dénomination Cactus. En variante, le capteur 46 peut être d'un autre type ou ne permettre de mesurer qu'un seul ou certains des paramètres mentionnés ci-dessus.

Le capteur 48 est un capteur d'indice de basicité ou BN, parfois dénommé indice d'alcalinité. Il peut s'agir d'un capteur fonctionnant avec la technologie infrarouge, dans le moyen infrarouge, ou de tout autre capteur adapté à la détermination du BN d'un lubrifiant.

L'installation 2 comprend également un premier capteur de niveau 54 et un deuxième capteur de niveau 56 qui permettent respectivement de détecter lorsque la quantité de l'huile dans le réservoir 26 atteint un premier niveau N1 ou un deuxième niveau N2. Les signaux électriques de sortie S54 et S56 des capteurs 54 et 56 sont délivrés à l'unité 22.

En variante, les capteurs 54 et 56 peuvent être remplacés par un unique capteur, tel qu'un capteur de pression, qui permet de détecter lorsque l'huile atteint chacun des deux niveaux N1 et N2 dans le réservoir 26.

Les figures 2 à 5 illustrent schématiquement les étapes successives d'un procédé automatisé mis en oeuvre grâce à l'installation 2 de la figure 1. Ce procédé est automatisé en ce sens qu'il est peut être implémenté, partiellement ou de préférence totalement, sans intervention humaine, sous le contrôle de l'unité 22. Il en va de même pour le procédé explicité ci-après au sujet du deuxième mode de réalisation de l'invention.

Par défaut, et en dehors des phases de prélèvement, l'huile sortant du moteur s'écoule dans la conduite 4, dans le sens de la flèche F1 à la figure 1, du moteur M vers le bac de récupération 6, sans être retenue par la vanne 20 qui est en configuration ouverte ou passante, alors que les autres vannes sont fermées.

Lorsqu'il convient de déterminer l'indice de basicité de l'huile sortant du moteur M, l'unité 22 pilote la vanne 20 à la fermeture, de sorte qu'il est créé dans la conduite 4 une retenue où s'accumule une quantité d'huile, c'est-à-dire de lubrifiant, comme représenté par la partie grisée L à la figure 2.

Dans la configuration de la figure 2, la conduite 4 sert de colonne de décantation et des impuretés 1 s'accumulent au voisinage de la vanne 20, à l'intérieur de la conduite 4 et en partie basse de la quantité de lubrifiant L.

Dans cette première étape représentée par la configuration de la figure 2, les vannes 32 et 40 sont ouvertes, alors que les vannes 36 et 44 sont fermées.

Lorsque le niveau de lubrifiant L dans la conduite ou colonne 4 atteint l'embouchure 282, de l'huile commence à s'écouler à travers la première ligne de dérivation 28, plus particulièrement à travers le filtre 30 et la vanne 32, jusque dans le volume intérieur V26 du réservoir 26 dans lequel l'huile s'écoule par gravité. En effet, le débouché 284 de la première ligne 28 est situé en partie haute du réservoir 26 et l'huile peut s'écouler le long de la paroi du réservoir 26. Comme la vanne 44 est fermée, l'huile remplit progressivement la partie de la deuxième ligne d'évacuation 42 située en amont de la vanne 44, y compris les volumes internes des capteurs 46 et 48, puis le volume intérieur V26, en chassant l'air vers l'atmosphère, à travers la vanne 40. Cette étape correspond à la configuration représentée à la figure 3.

Lorsque le capteur 56 détecte que le niveau N2 d'huile à l'intérieur du réservoir 26 est atteint, l'unité 22 bascule l'installation 2 vers une nouvelle étape, représentée par la configuration de la figure 4, dans laquelle la vanne 20 passe en configuration ouverte, ce qui permet de vidanger la colonne de décantation en dirigeant le reliquat de la quantité L de lubrifiant présente en amont de la vanne 20 ainsi que les impuretés 1 vers le bac de récupération 6. L'écoulement dans le sens de la flèche F1 se poursuit donc jusque dans le bac 6. Par ailleurs, les vannes 32 et 40 sont fermées et la vanne 36 est ouverte, ce qui permet de mettre la partie du volume V26 qui n'est pas occupée par le lubrifiant, c'est-à-dire la partie de ce volume V26 située au-dessus du niveau N2, sous une pression d'air P1 égale à celle de la source d'air 12, qui, dans l'exemple, vaut 7 bars absolus.

Ceci étant fait, l'unité 22 fait passer l'installation 2 à une étape suivante, représentée par la configuration de la figure 5, où la vanne 44 est ouverte, les autres vannes conservant leur état de la configuration de la figure 4. Dans ce cas, la pression P1 de l'air dans la partie supérieure du volume V26 a pour effet de pousser l'huile dans la deuxième ligne d'évacuation 42, à travers les capteurs 46 et 48, ce qui permet à ces capteurs de fournir à l'unité 22 des signaux S46, respectivement S48, représentatifs des paramètres qu'ils ont détectés.

Le cas échéant, les signaux S46 et S48 peuvent être traités dans l'unité 22 pour déterminer les valeurs des paramètres contrôlés, notamment par comparaison avec des valeurs connues pour des lubrifiants de référence.

Les signaux S46 et S48, ou des signaux extrapolés à partir de ces signaux, peuvent être fournis à l'extérieur de l'installation 2 sous la forme d'un signal conjugué S2, exploitable par une unité centrale de contrôle du moteur M.

En pratique, la section de passage du capteur d'indice de basicité 48 est d'environ 3 mm par 0,1 mm et il convient de pouvoir alimenter cette section de passage avec un débit suffisant, pendant une durée suffisante à la réalisation de la mesure de l'indice de basicité. La construction de l'installation avec le réservoir 26 permet de créer une réserve formant un « tampon » d'huile, sous la forme de la quantité d'huile L1 contenue dans le réservoir 26 dans la configuration de la figure 4. Une partie de cette réserve d'huile L1 peut être déversée, de façon continue ou séquentielle, dans la deuxième ligne d'évacuation 42 pour que le capteur 48 dispose d'une quantité suffisante d'huile à analyser.

A partir de la configuration de la figure 5, il est possible, dans une étape subséquente, de poursuivre la vidange du réservoir 26 et de l'intégralité de la deuxième ligne d'évacuation 42 en maintenant la vanne 44 ouverte et en poursuivant l'injection d'air comprimé à travers la vanne 36.

En variante, il est possible d'arrêter la vidange du réservoir 26 lorsque le niveau d'huile atteint le niveau N1, de façon à conserver en permanence une quantité L2 d'huile dans la deuxième ligne d'évacuation 42, en particulier dans les capteurs 46 et 48 dont les parties actives au contact de l'huile ne risquent pas de sécher. Si cette deuxième approche est sélectionnée, une certaine quantité d'huile doit être utilisée lors d'une prochaine mesure, pour nettoyer préalablement la deuxième ligne d'évacuation 42 et ne pas perturber la prochaine mesure.

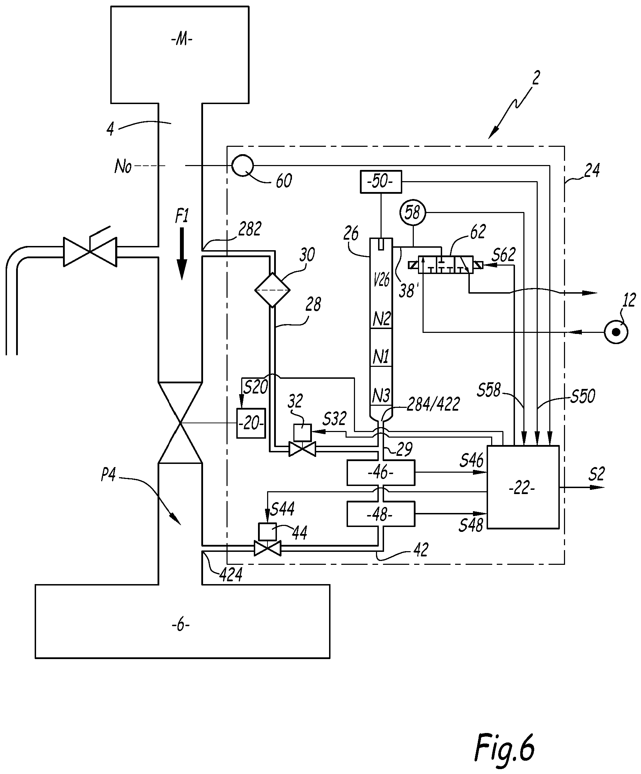

Dans les deuxième et troisième modes de réalisation de l'invention représentés aux figures 6 et suivantes, les éléments analogues à ceux du premier mode de réalisation portent les mêmes références. Dans ce qui suit, on décrit principalement ce qui distingue ces modes de réalisation du précédent.

Dans le mode de réalisation des figures 6 à 18, les première et deuxième lignes 28 et 42 se rejoignent au niveau d'un embranchement 29 en T. Ainsi, le débouché 284 de la première ligne de dérivation 28 est confondu avec l'embouchure 422 de la deuxième ligne d'évacuation 42. Le tronçon de ligne situé entre le réservoir 26 et l'embranchement 29 est commun aux première et deuxième lignes 28 et 42. Ce tronçon de ligne débouche en partie basse du réservoir 26, de sorte que l'huile qui s'écoule de la conduite 4 vers le réservoir 26 parvient directement en partie basse de ce réservoir.

On définit trois niveaux N1, N2 et N3 dans le réservoir 26, les niveaux N1 et N2 étant comparables à ceux du premier mode de réalisation.

Dans ce deuxième mode de réalisation, il n'est pas utilisé de capteurs de niveau identiques aux capteurs de niveaux 54 et 56, mais un capteur de pression 58 dont le signal de sortie S58 est fourni à l'unité de contrôle électronique 22. Par ailleurs, un capteur de niveau 60 est monté dans la conduite 4, en amont de la vanne 20, c'est-à-dire au-dessus de celle-ci.

En outre, les piquages 34 et 38 et les vannes 36 et 40 du premier mode de réalisation sont remplacés par un unique piquage 38' sur lequel est branché le capteur de pression 58, ainsi qu'un distributeur 62 à trois voies et trois orifices, qui est raccordé d'une part à la source d'air sous pression 12 et d'autre part à l'atmosphère ambiante. Le distributeur 62 est commandé par l'unité 22 au moyen d'un signal électrique dédié S62.

L'installation 2 comprend également un troisième capteur 50 monté en partie supérieure du réservoir 26 et disposé pour viser l'interface I26 entre une quantité de lubrifiant présente dans le réservoir 26 et l'air présent au-dessus de cette quantité. Le capteur 50 est un capteur utilisant la technologie de spectroscopie sur plasma induit par laser ou LIBS (de l'Anglais Laser Induced Breakdown Spectroscopy).

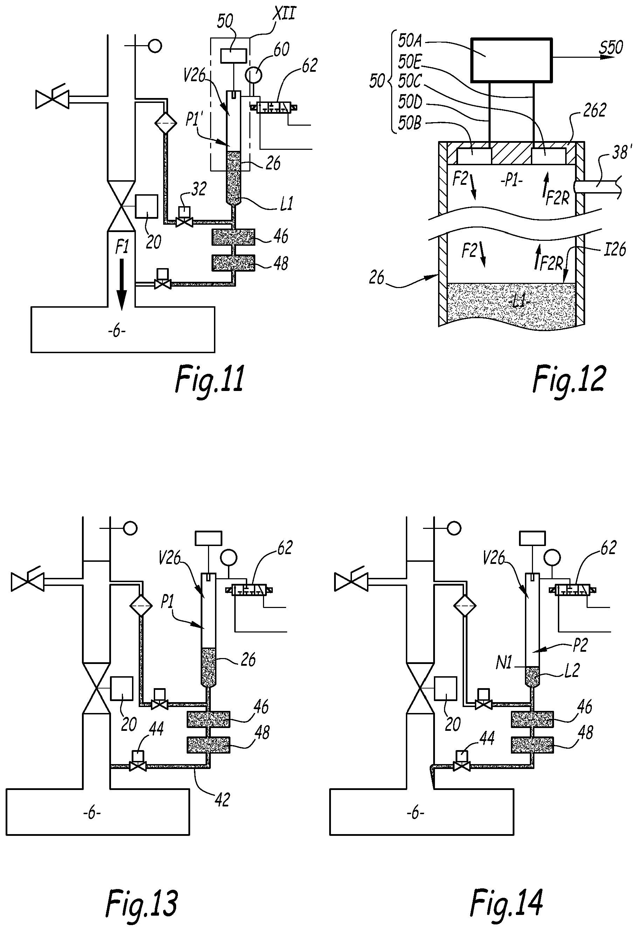

Plus précisément, comme visible à la figure 12, le capteur 50 comprend une unité de contrôle 50A, un émetteur 50B d'un faisceau laser dirigé vers l'interface I26, comme représenté par les flèches F2, ainsi qu'un récepteur 50C apte à recevoir un faisceau émis en retour, à partir de l'interface I26 et représenté par les flèches F2R. Le faisceau laser F2 émis par l'émetteur 50B excite la quantité L1 de lubrifiant et, lors de la désexcitation, il se produit une émission d'un spectre caractéristique de cette quantité L1, sous la forme du faisceau F2R émis en retour. Les composants 50B et 50C du capteur 50 sont intégrés à une paroi supérieure 262 du réservoir 26 et reliés à l'unité 50A par deux liaisons filaires 50D et 50E.

Cette technologie permet au capteur 50 de déterminer la teneur en fer dissout de l'huile contenue dans le réservoir 26, plus particulièrement la teneur en ions Fe²⁺ et Fe³⁺. Ceci permet de déterminer le niveau de corrosion des parties du moteur au contact avec l'huile et, par voie de conséquence, d'initier des actions de maintenance préventive ou corrective lorsque nécessaire.

En variante, un autre type de capteur 50, permettant également de déterminer la teneur en fer dissout de l'huile contenue dans le réservoir 26, peut être utilisé. Dans ce cas, ce capteur peut être intégré à la deuxième ligne 42, notamment disposé en aval du capteur d'indice de basicité 48.

Le fonctionnement de l'installation 2 est le suivant :
Par défaut, la vanne 20 est ouverte et les vannes 32 et 44 sont fermées, alors que le distributeur 62 est dans la configuration représentée à la figure 6 où il isole le volume intérieur V26 du réservoir 26 de la source 12 d'air comprimée et de l'atmosphère ambiante.

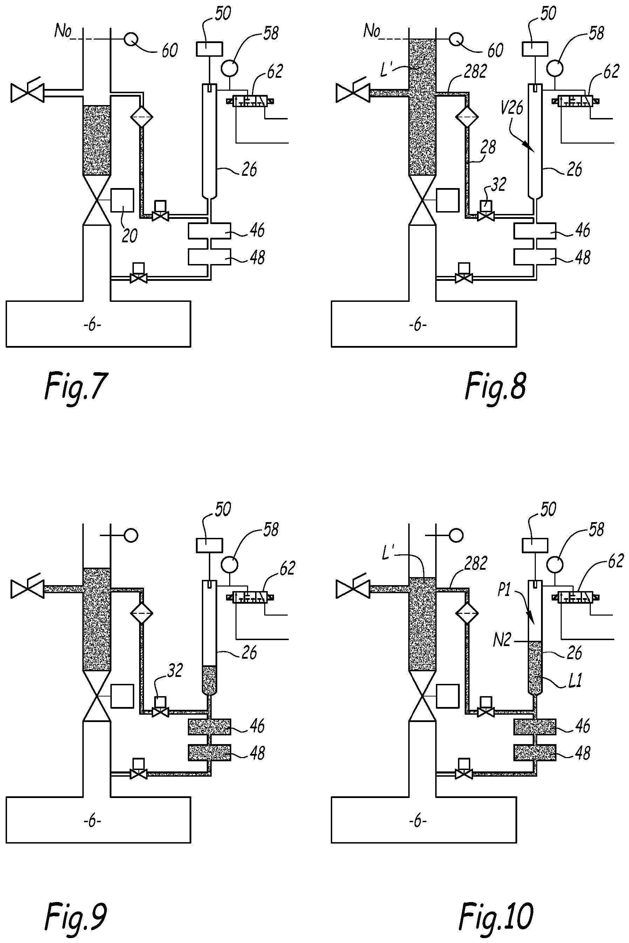

Lorsqu'il convient de procéder à la détermination de l'indice de basicité de l'huile sortant du moteur M, l'unité 22 active la vanne 20 au moyen du signal S20 dans une première étape, pour l'amener en configuration fermée représentée à la figure 7. Dans cette configuration, de l'huile est présente dans la première ligne de dérivation 28, entre le filtre 30 et la vanne 32, du fait d'une opération de décolmatage du filtre 30, effectuée précédemment et qui est explicité ci-après.

Dans cette configuration, les vannes 32 et 44 et le distributeur 62 sont fermés.

Le capteur de niveau 60 est positionné pour que, lorsque la colonne d'huile retenue dans la conduite 4 en amont de la vanne 20 atteint le niveau N0 détecté par ce capteur, comme représenté à la figure 8, une quantité prédéterminée de lubrifiant L' est présente au-dessus de l'embouchure 282. Par exemple, la quantité prédéterminée peut être égale à 100 ml. Lorsque le capteur de niveau 60 détecte que ce niveau N0 est atteint dans la conduite 4, le volume intérieur V26 du réservoir 26 est mis à la pression atmosphérique en actionnant le distributeur 62 pour l'amener dans la configuration de la figure 8.

A partir de cette configuration, l'unité 22 commande la vanne 32 et le distributeur 62 dans une étape suivante pour réaliser le transfert de la quantité L' d'huile de la conduite 4 vers le réservoir 26, comme représenté par la configuration de la figure 9. Dans cette configuration, la vanne 32 est ouverte, alors que le distributeur 62 est fermé. Le transfert d'huile de la conduite 4 vers le réservoir tampon 26 s'accompagne donc d'une augmentation de la pression d'air à l'intérieur du réservoir 26. Le taux de compression de l'air piégé dans le réservoir peut être relié, après étalonnage, au volume initial d'air dans le réservoir 26 et au volume d'huile transvasé.

Par exemple, pour une compression adiabatique et un volume d'air initial dans le réservoir 26 égal à 160 ml, la pression dans le réservoir 26 atteint 1,7 bar absolu pour 50 ml d'huile transférés.

De même, en considérant un réservoir 26 contenant initialement 250 ml d'air, il est possible de transférer 80 ml, soit la quantité L1 représentée à la figure 10, dans le réservoir 26 avant d'atteindre en partie supérieure de celui-ci une pression d'air P1 égale à 1,7 bar absolu. C'est l'exemple considéré dans ce qui suit.

Dans ce cas, le niveau d'huile N2 est atteint dans le réservoir 26 au niveau de l'étape représentée par l'installation 2 dans la configuration de la figure 10.

L'unité 22 commande alors automatiquement les vannes et le distributeur pour atteindre la configuration de la figure 11 où le réservoir 26 est mis sous pression à travers le distributeur 62 qui raccorde le volume V26 à la source d'air comprimé 12, de sorte que la pression P1' d'air à l'intérieur du réservoir 26 devient égale à 7 bars. Pour que ceci puisse avoir lieu, la vanne 32 a préalablement été basculée par l'unité 22 en configuration fermée, afin d'éviter un retour d'huile du réservoir 26 vers la canalisation 4. Par ailleurs, dans cette étape, la vanne 20 est basculée par l'unité 22 en configuration ouverte, de sorte que l'écoulement d'huile du moteur M vers le bac de récupération 6 peut à nouveau avoir lieu dans le sens de la flèche F1. Toujours dans cette étape, le capteur 50 est utilisé pour mesurer la teneur en fer dissout, notamment en ions Fe²⁺ et Fe³⁺, de la quantité L1 d'huile présente dans le réservoir 26.

Pour ce faire, le capteur 50 vise l'interface huile/air I26 qui se situe au niveau N2 dans le réservoir 26. Le signal de sortie S50 du capteur 50, ou un signal extrapolé à partir de ce signal, est intégré au signal de sortie S2 de l'installation 2.

A partir de cette configuration, l'unité 22 contrôle le distributeur 62 et la vanne 44 au moyen des signaux S62 et S44, respectivement pour fermer le distributeur 62 et ouvrir la vanne 44 et atteindre ainsi la configuration de la figure 13 où l'huile contenue dans le réservoir 26 est progressivement chassée de celui-ci du fait de la pression P1 régnant en partie supérieure du volume intérieur V26.

L'huile s'écoule donc à travers les capteurs 46 et 48 qui sont capables de détecter les paramètres pour lesquels ils sont prévus et de fournir des signaux correspondants, S46 et S48, à l'unité 22, comme dans le premier mode de réalisation.

La décharge de l'huile contenue dans le réservoir 26 à travers la deuxième ligne d'évacuation 42 peut avoir lieu en plusieurs cycles, par détentes successives du volume d'air piégé dans le réservoir et connexions successives à la source d'air 12. Pour un réservoir de 250 ml contenant initialement 80ml d'huile, on peut par exemple effectuer trois détentes successives, entre 7 bars et 6,2 bars, précédées de trois connexions à la source d'air 12. Ceci permet de délivrer un volume total de 50 ml dans la deuxième ligne d'évacuation 42 et d'atteindre la configuration de la figure 14 où une quantité résiduelle L2, de 30 ml de lubrifiant, demeure dans le réservoir 26 en étant soumise à une pression P2 égale à 6,2 bars.

Les trois détentes successives ont lieu en remplissant préalablement et successivement le réservoir 26 avec de l'air à 7 bars, au moyen d'une commande appropriée du distributeur 62.

Ces trois détentes permettent de faire circuler 50 ml de lubrifiant dans les capteurs 46 et 48 en trois étapes successives, ce qui leur permet de générer trois jeux de signaux S46 et S48 ou un jeu de signaux combinés, destiné(s) à et fourni(s) à l'unité 22, puis transmis et/ou traité(s) comme dans le premier mode de réalisation.

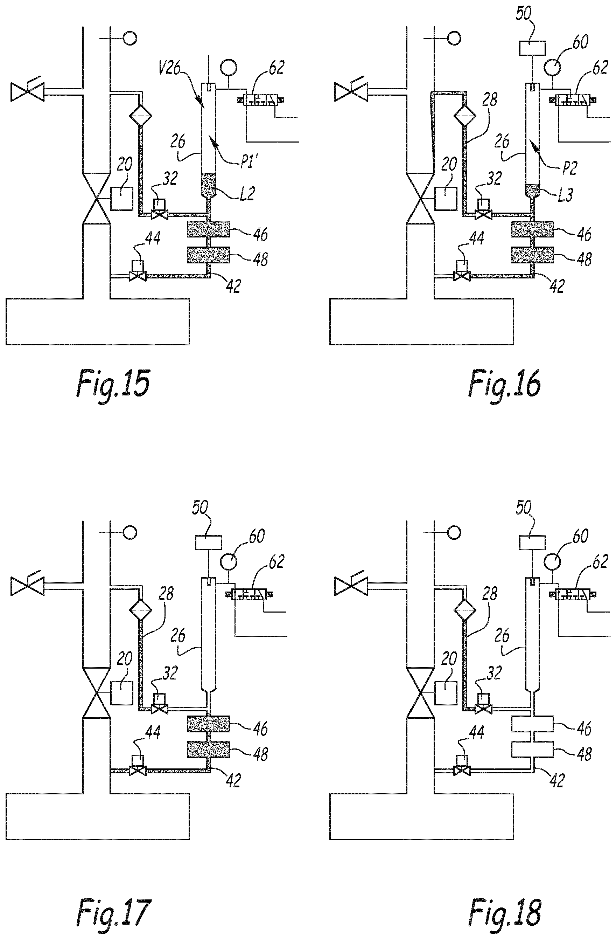

A partir de la configuration de la figure 14, l'unité 22 fait passer l'installation 2 dans la configuration de la figure 15 où le volume intérieur V26 du réservoir 26 est à nouveau pressurisé à la pression P1' de 7 bars, moyennant une commande appropriée du distributeur 62, alors que la vanne 44 est fermée.

Une fois cette opération réalisée, l'unité 22 commande la vanne 32 à l'ouverture et le distributeur 62 à la fermeture, ce qui a pour effet de chasser l'huile présente en partie basse du réservoir 26 à travers la première ligne de dérivation 28, jusque dans la conduite 4, dans un sens de décolmatage du filtre 30. Cette étape est représentée par la configuration de la figure 16. Le fait de faire baisser la pression dans le réservoir 26 de 7 à 6,2 bars permet de faire circuler une quantité d'environ 20 ml du réservoir 26 vers la conduite 4. Au terme de cette étape, il reste une quantité L3 égale à 10 ml de lubrifiant dans le réservoir 26, sous une pression P2 de 6,2 bars.

Une fois cette opération de décolmatage du filtre réalisé, l'unité 22 fait passer l'installation 2 dans la configuration de la figure 17 où la vanne 32 est à nouveau fermée, alors que la vanne 44 est ouverte et que le distributeur 62 est placé dans une configuration d'alimentation du volume V26 en air sous pression. Ceci a pour effet d'évacuer la quantité résiduelle d'huile présente dans la deuxième ligne d'évacuation 42 et dans les capteurs 46 et 48 jusqu'à obtenir la configuration de la figure 18 où la deuxième ligne d'évacuation 42 et les capteurs 46 et 48 sont vides d'huile et remplis d'air. Ceci correspond à la configuration de la figure 7 mentionnée ci-dessus.

On remarque sur les figures 17 et 18 que la partie de la première ligne de dérivation 28 située entre la vanne 32 et le débouché 284 est vidée par l'air provenant du réservoir 26. Ceci est à rapprocher du fait que, en pratique, la vanne 32 est disposée immédiatement en amont de l'embranchement 29.

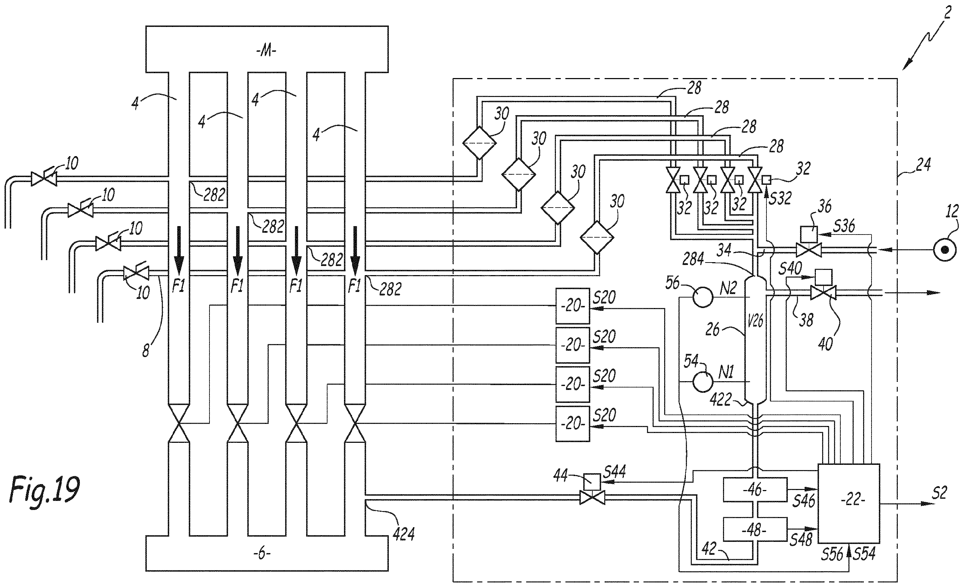

Dans le troisième mode de réalisation de l'invention représenté à la figure 19, plusieurs conduites 4 sont utilisées, chacune d'entre elle étant prévue pour collecter de l'huile à partir d'un unique cylindre du moteur M.

Chaque conduite 4 est équipée d'une vanne 20 commandée par l'unité électronique 22 et qui permet d'interrompre l'écoulement d'un flux F1 de lubrifiant dans la conduite 4 considérée. Une première ligne de dérivation 28 est raccordée, d'une part, à chaque conduite 4, en amont de sa vanne 20 et, d'autre part, à l'entrée du réservoir tampon 26 qui est le même que dans le premier mode de réalisation. L'installation 2 comprend donc autant de premières lignes de dérivation 28 que de conduites 4. En partant de son embouchure 282, chaque première ligne de dérivation 28 est équipée d'un filtre 30 et d'une vanne d'arrêt 32. Les quatre premières lignes 28 se rejoignent en aval de leurs vannes d'arrêt 32 respectives et le piquage 34 est commun aux quatre premières lignes de dérivation 28, de même que leur débouché 284 dans le réservoir tampon 26.

Un piquage 8 est prévu sur chaque conduite 4 et équipé d'une vanne 10 commandée manuellement, selon une approche parallèle à celle mentionnée ci-dessus au sujet du premier mode de réalisation. En variante, seule une ou certaines conduites 4 et/ou sont équipées d'un tel piquage 8.

La deuxième ligne d'évacuation 42 est commune pour tous les cylindres du moteur et reçoit, en aval du réservoir 26, l'huile provenant de toutes les premières lignes de dérivation 28. Le débouché 424 de la deuxième ligne d'évacuation est disposé sur l'une des conduites 4, en aval de sa vanne d'arrêt 20.

Ce troisième mode de réalisation permet d'optimiser l'encombrement des conduites 4 et leur cheminement au sein du compartiment moteur d'un navire. Elle permet un gain de place par rapport au premier mode de réalisation.

En mettant en oeuvre successivement la méthode explicitée ci-dessus au sujet du premier mode de réalisation, pour chacune des conduites 4, l'installation de ce troisième mode de réalisation permet de connaître, grâce au capteur 48, identique à celui du premier mode de réalisation, l'indice de basicité du carburant en sortie de chacun des cylindres du moteur M sur lequel est raccordée une conduite 4.

Dans l'exemple de la figure 19, quatre conduites 4 sont prévues, chacune dédiée à un cylindre du moteur M. En variante, le nombre de conduites 4 est différent, tout en restant supérieur ou égal à 2, afin d'adapter l'installation 2 en fonction de la configuration du moteur M et de la place disponible pour loger les conduites 4.

Quel que soit le mode de réalisation, l'installation 2 permet une mesure efficace de l'indice de basicité ou BN d'une huile sortant du moteur M grâce à un procédé qui peut être automatisé et qui ne nécessite pas de connaissance particulière de la part d'un utilisateur, puisque le signal S2 peut être directement lisible, soit par l'homme soit par une machine.

En pratique, la pression maximum P1' régnant dans le volume intérieur V26 du réservoir 26, qui dépend de la pression de la source 12, n'est pas limitée à 7 bars. Elle est comprise entre 6 et 12 bars, de préférence entre 7 et 10 bars en fonction de la pression du réseau d'air comprimé disponible sur le navire. La valeur de 7 bars est préférée car elle donne de bons résultats expérimentaux et correspond à un niveau de pression couramment disponible. Il importe que cette pression P1 soit supérieure à la pression P4 de l'huile dans la conduite 4, qui est comprise entre 1,1 et 6 bars comme mentionné ci-dessus. En effet, c'est la différence entre les pressions P1 et P4 qui assure l'écoulement de l'huile à travers la deuxième conduite d'évacuation 42.

Quel que soit le mode de réalisation, l'installation 2, qui est pour l'essentiel comprise dans le boîtier 24, est facile à installer à bord d'un navire et ne nécessite pas la mise en place de la vanne 20 dans la conduite 4, le branchement des lignes 28 et 42 sur cette conduite et son alimentation en courant et en air sous pression. L'installation 2 peut donc être aisément implantée sur un navire neuf ou utilisée pour rétrofiter un navire en service.

L'invention est décrite ci-dessus dans le cas de son utilisation pour un moteur de propulsion de navire. Elle est toutefois applicable à d'autres équipements, par exemple un moteur auxiliaire ou d'accessoire de navire, ainsi qu'une boîte à vitesses, notamment une boîte à vitesse d'hydrolienne ou d'éolienne.

Dans ce qui précède, les mots « huile » et « lubrifiant » sont utilisés indistinctement car une huile moteur est un lubrifiant. L'invention est toutefois applicable à d'autres lubrifiants tels que les huiles pour transmissions et pour engrenages, les huiles pour compresseurs, les huiles hydrauliques, les huiles de turbine ou encore les huiles pour centrifugeuse.

Les caractéristiques des modes de réalisation et variantes envisagées ci-dessus peuvent être combinées pour générer de nouveaux modes de réalisation de l'invention.

## Revendications

1. Installation (2) de suivi de l'évolution de la basicité (BN) d'un lubrifiant circulant dans un équipement (M), cette installation comprenant :
- au moins une conduite (4) de circulation (F1) du lubrifiant, cette conduite étant raccordée, en amont, à l'équipement et, en aval, à un bac de récupération (6)
- au moins un capteur (48) de détermination de l'indice de basicité (BN) du lubrifiant
- un réservoir (26) d'accumulation du lubrifiant
- une première ligne (28) de dérivation raccordée, d'une part, à la conduite et, d'autre part, au réservoir
- une deuxième ligne (42) d'évacuation du lubrifiant, du réservoir vers le bac de récupération, cette deuxième ligne étant disposée en aval de la première ligne de dérivation (28),
- une première vanne (20) d'interruption commandée de la circulation (F1) du lubrifiant dans la conduite (4),
- une deuxième vanne (32) d'interruption commandée de la circulation du lubrifiant dans la première ligne de dérivation, dans laquelle ledit au moins un capteur (48) est disposé sur la deuxième ligne (42) d'évacuation, l'installation étant **caractérisée en ce que** :
- le réservoir (26) est un réservoir tampon (26) d'accumulation du lubrifiant
- une troisième vanne (44) d'interruption commandée de la circulation du lubrifiant est comprise dans la deuxième ligne d'évacuation
- le capteur (48) permet de déterminer l'indice de basicité du lubrifiant en sortie du réservoir tampon (26)
- -la première ligne (28) de dérivation est raccordée à la conduite en amont de la première vanne.

2. Installation selon la revendication 1, **caractérisée en ce qu'**elle comprend des moyens (12, 22, 36, 40 ; 12, 22, 62) de mise sous pression gazeuse du volume intérieur (V26) du réservoir tampon (26).

3. Installation selon la revendication 2, **caractérisée en ce que** les moyens de mise sous pression gazeuse comprennent une source d'air comprimé (12) et un jeu de vannes (36,40) ou un distributeur pneumatique (62) de mise en communication sélective du volume intérieur (V26) du réservoir tampon (26) avec la source d'air comprimé ou l'atmosphère ambiante.

4. Installation selon l'une des revendications précédentes, **caractérisée en ce qu'**elle comprend des moyens (54, 56 ; 60) de détection du niveau de lubrifiant dans le réservoir tampon (26).

5. Installation selon les revendications 2 et 4, **caractérisée en ce que** les moyens de détection du niveau de lubrifiant dans le réservoir comprennent un capteur (58) de la pression gazeuse dans le volume intérieur (V26) du réservoir tampon (26).

6. Installation selon l'une des revendications précédentes, **caractérisée en ce qu'**elle comprend, en outre,
- un capteur (46) de densité (D), viscosité (V), humidité (H) et température (T) disposé également sur la deuxième ligne d'évacuation (42)
- un capteur (50) de la teneur en fer dissous du lubrifiant présent dans le réservoir tampon.

7. Procédé automatisé de suivi de l'évolution de la basicité d'un lubrifiant circulant dans un équipement (M), au moyen d'une installation (2) selon l'une des revendications précédentes, **caractérisé en ce qu'**il comprend au moins des étapes consistant à :
a) fermer la première vanne (20),
b) ouvrir la deuxième vanne (32) et fermer la troisième vanne (44) pour alimenter le réservoir tampon à partir d'une quantité (L ; L') de lubrifiant accumulée dans la conduite (4), en amont de la première vanne,
c) ouvrir la troisième vanne (44) pour faire circuler le lubrifiant présent dans le réservoir tampon à travers la deuxième ligne d'évacuation (42), au contact du capteur (48) de détermination de l'indice de basicité du lubrifiant,
d) utiliser un signal de sortie (S48) de ce capteur pour déterminer l'indice d'alcalinité du lubrifiant.

8. Procédé selon la revendication 7, **caractérisé en ce qu'**il est mis en oeuvre avec une installation (2) selon l'une des revendications 2 ou 3 et **en ce qu'**il comprend une étape e) postérieure à l'étape b) et antérieure à l'étape c) et consistant à :
e) mettre en pression gazeuse le volume intérieur (V26) du réservoir tampon (26), avec une pression (P1) comprise entre 6 et 12 bars, de préférence entre 7 et 10 bars, de préférence encore égale à 7 bars.

9. Procédé selon l'une des revendications 7 ou 8, **caractérisé en ce que** l'étape c) est interrompue, alors qu'une quantité résiduelle (L2) de lubrifiant demeure dans le réservoir tampon (26).

10. Procédé selon la revendication 9, **caractérisé en ce qu'**il comprend une étape f) postérieure à l'étape d) et consistant à :
f) décolmater un filtre (30) intégré à la première ligne de dérivation (28), en faisant circuler du lubrifiant du réservoir tampon (26) vers la conduite (4).

11. Méthode de suivi du fonctionnement d'un équipement (M) embarqué sur un navire, **caractérisée en ce qu'**elle comprend la détermination, à bord du navire, de l'indice de basicité (BN) d'un lubrifiant de l'équipement par la mise en oeuvre d'un procédé selon l'une des revendications 7 à 10.

## Patentansprüche

1. Anlage (2) zur Überwachung der Änderung der Basizität (BN) eines Schmiermittels, das in einer Ausrüstung (M) zirkuliert, wobei diese Anlage umfasst:
- mindestens eine Leitung (4) für die Zirkulation (F1) des Schmiermittels, wobei diese Leitung stromaufwärts an die Ausrüstung und stromabwärts an ein Auffangbecken (6) angeschlossen ist,
- mindestens einen Sensor (48) zur Bestimmung des Basizitätsindex (BN) des Schmiermittels,
- einen Tank (26) zur Speicherung des Schmiermittels,
- eine erste Bypassleitung (28), die einerseits an die Leitung und andererseits an den Tank angeschlossen ist,
- eine zweite Leitung (42) zum Auslassen des Schmiermittels aus dem Tank in den Auffangbehälter, wobei diese zweite Leitung stromabwärts der ersten Bypassleitung (28) angeordnet ist,
- ein erstes Ventil (20) zur gesteuerten Unterbrechung der Zirkulation (F1) des Schmiermittels in der Leitung (4),
- ein zweites Ventil (32) zur gesteuerten Unterbrechung der Zirkulation des Schmiermittels in der ersten Bypassleitung, wobei der mindestens eine Sensor (48) auf der zweiten Auslassleitung (42) angeordnet ist, wobei die Anlage **dadurch gekennzeichnet ist, dass**:
- der Tank (26) ein Puffertank (26) zur Speicherung des Schmiermittels ist,
- ein drittes Ventil (44) zur gesteuerten Unterbrechung der Zirkulation des Schmiermittels in der zweiten Auslassleitung inbegriffen ist,
- der Sensor (48) gestattet, den Basizitätsindex des Schmiermittels am Ausgang des Puffertanks (26) zu bestimmen,
- die erste Bypassleitung (28) an der Leitung stromaufwärts des ersten Ventils angeschlossen ist.

2. Anlage nach Anspruch 1, **dadurch gekennzeichnet, dass** sie Mittel (12, 22, 36, 40; 12, 22, 62) umfasst, um das Innenvolumen (V26) des Puffertanks (26) unter Gasdruck zu setzen.

3. Anlage nach Anspruch 2, **dadurch gekennzeichnet, dass** die Mittel zur Gasdruckbeaufschlagung eine Druckluftquelle (12) und einen Satz von Ventilen (36, 40) oder einen pneumatischen Verteiler (62) umfassen, um das Innenvolumen (V26) des Puffertanks (26) selektiv mit der Druckluftquelle oder der umgebenden Atmosphäre in Kommunikation zu versetzen.

4. Anlage nach einem der vorhergehenden Ansprüche, **dadurch gekennzeichnet, dass** sie Mittel (54, 56; 60) zum Erfassen des Schmiermittelstandes in dem Puffertank (26) umfasst.

5. Anlage nach den Ansprüchen 2 und 4, **dadurch gekennzeichnet, dass** die Mittel zum Erfassen des Schmiermittelstandes in dem Puffertank einen Sensor (58) für den Gasdruck in dem Innenvolumen (V26) des Puffertanks (26) umfassen.

6. Verfahren nach einem der vorhergehenden Ansprüche, **dadurch gekennzeichnet, dass** es ferner umfasst:
- einen Sensor (46) für Dichte (D), Viskosität (V), Feuchtigkeit (H) und Temperatur (T), der ebenfalls auf der zweiten Auslassleitung (42) angeordnet ist,
- einen Sensor (50) für den Gehalt an gelöstem Eisen in dem Schmiermittel, das sich in dem Pufferbehälter befindet.

7. Automatisiertes Verfahren zur Überwachung der Änderung der Basizität in einem in einer Ausrüstung (M) zirkulierenden Schmiermittel mittels einer Anlage (2) nach einem der vorhergehenden Ansprüche, **dadurch gekennzeichnet, dass** es mindestens die folgenden Schritte umfasst:
a) Schließen des ersten Ventils (20),
b) Öffnen des zweiten Ventils (32) und Schließen des dritten Ventils (44), um den Puffertank aus einer in der Leitung (4) stromaufwärts des ersten Ventils gespeicherten Schmiermittelmenge (L; L') zu versorgen,
c) Öffnen des dritten Ventils (44), um das in dem Pufferbehälter befindliche Schmiermittel durch die zweite Auslassleitung (42) im Kontakt mit dem Sensor (48) zur Bestimmung des Basizitätsindex des Schmiermittels zirkulieren zu lassen,
d) Verwenden eines Ausgangssignals (S48) dieses Sensors, um den Alkalinitätsindex des Schmiermittels zu bestimmen.

8. Verfahren nach Anspruch 7, **dadurch gekennzeichnet, dass** es mit einer Anlage (2) nach einem der Ansprüche 2 oder 3 durchgeführt wird und dass es einen Schritt e) nach Schritt b) und vor Schritt c) umfasst und darin besteht:
e) das Innenvolumen (V26) des Puffertanks (26) mit einem Druck (P1) zwischen 6 und 12 bar, vorzugsweise zwischen 7 und 10 bar, noch bevorzugter von gleich 7 bar, unter Gasdruck zu setzen.

9. Verfahren nach einem der Ansprüche 7 oder 8, **dadurch gekennzeichnet, dass** Schritt c) abgebrochen wird, während eine Restmenge (L2) an Schmiermittel im Puffertank (26) verbleibt.

10. Verfahren nach Anspruch 9, **dadurch gekennzeichnet, dass** es einen Schritt f) nach Schritt d) umfasst und darin besteht:
f) Abreinigen eines in der ersten Bypassleitung (28) integrierten Filters (30), indem Schmiermittel aus dem Puffertank (26) in die Leitung (4) geleitet wird.

11. Methode zur Überwachung des Betriebs einer Ausrüstung (M), die sich an Bord eines Schiffes befindet, **dadurch gekennzeichnet, dass** sie die Bestimmung des Basizitätsindex (BN) in einem Schmiermittel der Ausrüstung an Bord des Schiffes durch die Durchführung eines Verfahrens nach einem der Ansprüche 7 bis 10 umfasst.

## Claims

1. An installation (2) for following up the evolution of the basicity (BN) of a lubricant circulating in a piece of equipment (M), this installation comprising:
- at least one conduit (4) for circulating (F1) the lubricant, this conduit being connected upstream to the piece of equipment and downstream to a recovery pan (6),
- at least one sensor (48) for determining the basicity index (BN) of the lubricant,
- a tank (26) for accumulating the lubricant,
a first bypass line (28) connected to the conduit, on the one hand, and to the tank, on the other hand,
a second line (42) for discharging the lubricant, from the tank to the recovery pan, this second line being positioned downstream from the first bypass line (28),
- a first controlled valve (20) for interrupting the circulation (F1) of the lubricant in the conduit (4),
- a second controlled valve (32) for interrupting the circulation of the lubricant in the first bypass line,
wherein the at least one sensor (48) is positioned on the second line (42) for discharging the installation being **characterized in that**:
- the tank (26) is a buffer tank (26) for accumulating the lubricant,
-- a third controlled valve (44) for interrupting the circulation of the lubricant is comprised in the second line for discharging
- the sensor (48) allows determining the basicity index of the lubricant at the outlet of the buffer tank (26)
- the first bypass line (28) is connected to the conduit upstream of the first valve.

2. The installation according to claim 1, **characterized in that** it comprises means (12, 22, 36, 40; 12, 22, 62) for gas pressurization of the inner volume (V26) of the buffer tank (26).

3. The installation according to claim 2, **characterized in that** the gas pressurization means comprise a source of compressed air (12) and a set of valves (36, 40) or a pneumatic distributor (62) for selectively putting the inner volume (V26) of the buffer tank (26) in communication with a source of compressed air or the ambient atmosphere.

4. The installation according to one of the preceding claims, **characterized in that** it comprises means (54, 56; 60) for detecting the lubricant level in the buffer tank (26).

5. The installation according to claims 2 and 4, **characterized in that** the means for detecting the lubricant level in the tank comprise a gas pressure sensor (58) in the inner volume (V26) of the buffer tank (26).

6. The installation according to one of the preceding claims, **characterized in that** it further comprises,
- a sensor (46) for measuring density (D), viscosity (V), humidity (H) and temperature (T) also positioned on the second discharge line (42),
- a sensor (50) for measuring the dissolved iron content of the lubricant present in the buffer tank.

7. An automated method for following the evolution of the basicity of a lubricant circulating in a piece of equipment (M), by means of an installation (2) according to one of the preceding claims, **characterized in that** it comprises at least steps of:
a) closing the first valve (20),
b) opening the second valve (32) and closing the third valve (44) for supplying the buffer tank from an amount (L; L') of lubricant accumulated in the conduit (4), upstream from the first valve,
c) opening the third valve (44) for circulating the lubricant present in the buffer tank through the second discharge line (42), in contact with the sensor (48) for determining the basicity index of the lubricant,
d) using an output signal (S48) of this sensor for determining the alkalinity of the lubricant.

8. The method according to claim 7, **characterized in that** it is applied with an installation (2) according to one of claims 2 or 3, and **in that** it comprises a step e) after step b) and before step c) and consisting of:
e) gas pressurizing the inner volume (V26) of the buffer tank (26), with a pressure (P1) comprised between 6 and 12 bars, preferably between 7 and 10 bars, still preferably 7 bars.

9. The method according to one of claims 7 or 8, **characterized in that** step c) is interrupted, while a residual amount (L2) of lubricant remains in the buffer tank (26).

10. The method according to claim 9, characterizing that it comprises a step f) after step d) and consisting of:
f) unclogging a filter (30) integrated to the first bypass line (28) by having the lubricant circulate from the buffer tank (26) to the conduit (4).

11. A method for following up the operation of a piece of equipment (M) on board a ship, **characterized in that** it comprises the determination, onboard the ship, of the basicity index (BN) of a lubricant of the piece of equipment by applying a method according to one of claims 7 to 10.
